# FASCICULE DE BREVET EUROPEEN

(11) **EP 2 386 263 B1**
(45) Date de publication et mention de la délivrance du brevet: **26.02.2014**
(21) Numéro de dépôt: 10162562.2
(22) Date de dépôt: 11.05.2010
(51) Int. Cl.: A61B 1/00, F21K 99/00, F21W 131/20, F21W 131/202, F21Y 101/02, A61B 1/06, A61B 1/24

(54) **Module d'éclairage à diode électroluminescente pour pièce à main chirurgicale ou dentaire**
Beleuchtungsmodul mit Elektrolumineszenzdioden für chirurgisches oder zahntechnisches Handstück
Lighting module with light-emitting diodes for a surgical or dental handpiece

(43) Date de publication de la demande: 16.11.2011
(73) Titulaire: Bien-Air Holding SA, 2500 Bienne 6 (CH)
(72) Inventeur: Waelti, Gabriel, 2605 Sonceboz-Sombeval (CH); Schönbächler, Edgar, 2000, Neuchâtel (CH)
(74) Mandataire: Ravenel, Thierry Gérard Louis

(56) Documents cités:
- EP-A1- 1 090 608
- US-A1- 2001 038 992
- US-A1- 2003 219 694
- US-A1- 2007 134 616

## Description

La présente invention concerne un dispositif d'éclairage à diode électroluminescente destiné à équiper une pièce à main à usage dentaire ou chirurgical. La présente invention concerne en particulier un dispositif d'éclairage permettant de remplacer dans une pièce à main existante une ampoule à filament par une diode électroluminescente sans besoin de modifier le câblage électrique de la pièce à main ou de l'unité (couramment appelée « unit ») qui commande la pièce à main et qui assure son alimentation en énergie et en fluides.

La plupart des instruments dentaires ou chirurgicaux sont équipés d'un dispositif d'éclairage comprenant une source lumineuse permettant d'éclairer la zone de travail de l'instrument, ce qui facilite singulièrement la tâche du praticien en lui permettant de jouir d'une bonne vision du champ opératoire. Jusqu'à il y a peu, la source lumineuse était constituée par une ampoule à filament dont le fonctionnement est éprouvé. Toutefois, depuis quelques temps déjà, la tendance des fabricants de pièces à main à usage dentaire ou chirurgical est de remplacer les ampoules à filament par des diodes électroluminescentes. Les avantages des diodes électroluminescentes par rapport aux ampoules à filament classiques sont connus : meilleur rendement (autrement dit, pour une même intensité d'éclairage, la consommation électrique d'une diode est moindre que celle d'une ampoule), durée de vie accrue et production d'une lumière plus proche de la lumière naturelle.

Afin de remplacer les ampoules à filament par des diodes électroluminescentes, diverses solutions ont été adoptées. La plus triviale de ces solutions consiste simplement à échanger l'ampoule à filament au profit d'une diode électroluminescente. Dans certains cas, cette solution ne fonctionne tout simplement pas. En effet, en-dessous d'une tension seuil, la diode ne s'éclaire pas. Par conséquent, il faut que l'électronique de la pièce à main ou de l'unité de commande et de fourniture de fluides à laquelle la pièce à main est raccordée soit capable de fournir à la diode une tension d'alimentation supérieure à la tension de seuil de cette diode, ce qui n'est pas toujours le cas. Par ailleurs, quand bien même la pièce à main ou l'unité de commande est capable de fournir à la diode électroluminescente une tension supérieure à sa tension seuil, on sait que le courant dans une diode augmente très rapidement en fonction de la tension. Par conséquent, sans contrôle de l'intensité du courant dans la diode, les risques de dépasser la valeur du courant acceptable par la diode et donc de détruire la diode sont importants.

Pour remédier à ce dernier problème, il a déjà été proposé de monter une résistance en série avec la diode électroluminescente. Cette solution a pour mérite de limiter le courant dans la diode et donc de limiter les risques de destruction de la diode dus à des courants trop intenses. Cependant, le fonctionnement de ce montage est tributaire des valeurs en tension et en intensité du courant fourni par la pièce à main ou par l'unit à laquelle la pièce à main est raccordée et n'est donc pas reproductible. Ainsi, en fonction de l'équipement dont dispose tel ou tel praticien, le fonctionnement de la diode électroluminescente est aléatoire, ce qui n'est pas acceptable.

Une autre solution encore consiste à intégrer dès l'origine dans la pièce à main un circuit de conversion pour l'alimentation en courant de la diode. Il s'agit cependant là d'une solution qui ne peut être implémentée dans un instrument existant et qui oblige le praticien à faire l'acquisition d'un instrument neuf, ce qui est coûteux d'un point de vue économique pour le praticien.

Le document EP 1 090 608 décrit une source de lumière comprenant une plaque de circuit imprimé sur la surface supérieure de laquelle est prévu un logement circulaire dans lequel sont logées une pluralité de diodes électroluminescentes. Les diodes électroluminescentes sont montées nues, c'est-à-dire non encapsulées. Par ailleurs, le logement est recouvert au moyen d'une résine époxy transparente. Finalement, des fils électriques sont reliés à des contacts de la plaque de circuit imprimé. Ce document omet de décrire que la source de courant et la diode électroluminescente sont encapsulées dans un même volume clos.

Le document US 2001/038992 décrit un dispositif lumineux comprenant un guide de lumière à proximité duquel est prévu un condenseur qui condense la lumière produite par une pluralité de diodes électroluminescentes montées selon une matrice de diodes. Chacune de ces diodes est reliée à un circuit de commande qui présente à ses bornes une tension prédéterminée transformée par une source d'alimentation et qui fournit à la diode considérée un courant d'alimentation. Ce document ne décrit pas un module d'éclairage comprenant une diode électroluminescente et une source de courant encapsulées dans un même volume clos.

La présente invention a pour but de pallier les inconvénients susmentionnés ainsi que d'autres encore en procurant un dispositif d'éclairage à diode électroluminescente qui garantisse un fonctionnement fiable et reproductible de la diode tout en pouvant être intégré dans une pièce à main existante en lieu et place d'une ampoule à filament.

A cet effet, la présente invention concerne un module d'éclairage à diode électroluminescente pour pièce à main à usage dentaire ou chirurgical, ce module d'éclairage comprenant une source de courant délivrant un courant de fonctionnement à une diode électroluminescente, caractérisé en ce que la source de courant et la diode électroluminescente sont encapsulées dans un même volume clos, le module d'éclairage comprenant des moyens de connexion électriques pour son raccordement à un réseau d'alimentation en électricité.

Grâce à ces caractéristiques, la présente invention procure un module d'éclairage à diode électroluminescente dont les éléments nécessaires au bon fonctionnement de la diode ainsi que la diode elle-même sont logés dans un même volume clos. On obtient donc un composant discret dont le fonctionnement est reproductible quelles que soient les caractéristiques électriques de la pièce à main dans laquelle il est intégré. Ce composant discret forme un élément consommable amovible et remplaçable avec un dispositif d'alimentation intégré dans le consommable et indissociable de la diode électroluminescente.

Selon une caractéristique complémentaire de l'invention, le module d'éclairage constitue une unité interchangeable pièce pour pièce avec une ampoule à filament du type utilisé dans les pièces à main existantes.

Selon encore une autre caractéristique de l'invention, le module d'éclairage est adapté en formes et en dimensions de même que du point de vue de ses moyens de connexion électriques pour pouvoir être substitué à une ampoule à filament utilisée dans les pièces à main existantes.

Grâce à ces autres caractéristiques, le module d'éclairage à diode électroluminescente selon l'invention peut avantageusement remplacer pièce pour pièce une ampoule à filament du commerce équipant une pièce à main existante. La présente invention permet ainsi avantageusement de porter les pièces à main existantes à un standard de qualité plus élevé en remplaçant une ampoule à filament par une diode électroluminescente sans que cela nécessite une quelconque modification mécanique ou électrique de ces pièces à main. L'utilisateur dispose donc d'une pièce à main dont le dispositif d'éclairage présente une plus grande durée de vie et produit un éclairage très proche de celui de la lumière naturelle sans être pour autant obligé de faire l'acquisition d'une pièce à main neuve. Il suffit en effet à l'utilisateur de faire l'acquisition du module d'éclairage selon l'invention et de le brancher en lieu et place de l'ampoule à filament équipant d'origine sa pièce à main. Comme le module d'éclairage occupe le même volume qu'une ampoule à filament et que ses connexions électriques sont disposées au même endroit que sur l'ampoule, le remplacement d'une telle ampoule par un module d'éclairage conforme à l'invention ne nécessite ni adaptations mécaniques, ni adaptations électriques.

Selon une première variante de réalisation de l'invention, la source de courant est alimentée depuis l'extérieur du volume clos par une source de tension et comprend des moyens pour convertir la tension fournie par la source de tension extérieure en courant de fonctionnement de la diode électroluminescente.

La source de tension peut être une source de tension variable. Dans ce cas, on utilise une source de courant dont le courant produit varie en fonction de la tension d'entrée. Ceci est nécessaire dans le cas où l'on souhaite que l'intensité de l'éclairage fourni par la diode soit d'autant plus élevée que la tension appliquée est grande.

Dans le cas où la source de tension utilisée est une source de tension variable, un dispositif de commande et de limitation du courant est raccordé sur la source de courant afin de définir un rapport de proportionnalité entre la valeur de la tension appliquée et l'intensité du courant fourni par la source de courant qui conditionne l'intensité de l'éclairage produit par la diode électroluminescente.

Selon une seconde variante de réalisation de l'invention, la source de courant est alimentée depuis l'extérieur du volume clos par une source de courant délivrant un courant d'intensité donnée et comprend des moyens pour faire varier le courant d'intensité donnée afin d'obtenir le courant de fonctionnement de la diode électroluminescente.

Selon une caractéristique complémentaire de l'invention, le module d'éclairage comprend, en amont de la source de courant, un élément redresseur grâce auquel la diode électroluminescente peut être montée sans considération de polarité.

Selon une autre caractéristique de l'invention, on intercale entre l'élément redresseur et la source de courant un élément de protection contre les surtensions. Cet élément a pour but de protéger le module d'éclairage selon l'invention contre les surtensions qui peuvent survenir à l'entrée du module d'éclairage en cas d'erreur d'utilisation de la part du praticien. En aval de la source de courant, on peut également prévoir un élément de protection en cas d'absence de charge. Cet élément de protection a pour but de protéger le module d'éclairage en cas d'absence de la diode. En effet, si la diode est manquante ou hors d'usage, la tension peut augmenter jusqu'à des valeurs correspondant à la destruction du module.

Selon encore une autre caractéristique de l'invention, on intercale entre l'élément redresseur et l'élément de protection contre les surtensions un premier filtre pour filtrer les interférences électromagnétiques générées par le fonctionnement de la pièce à main et qui perturbent la source de courant. En aval de la source de courant, après l'élément de protection contre les absences de charge, on peut prévoir un second filtre qui a pour but de stabiliser et lisser le courant produit par la source de courant et de filtrer les interférences inhérentes au bruit provoqué par le fonctionnement de la source de courant. Effectivement, cette source de courant est typiquement une source de courant à découpage fonctionnant à la fréquence de 1 Mhz.

Dans un mode de réalisation de l'invention, la diode électroluminescente et son module de commande associé sont introduits dans un tube métallique, plastique ou céramique qui définit un volume hermétiquement clos, le tube étant fermé à sa base par une embase et comprenant à son extrémité supérieure, du côté de la diode électroluminescente, une lentille de collimation collée ou sertie sur le tube.

Une autre technique d'encapsulation du module d'éclairage selon l'invention consiste à se munir d'un conteneur dans lequel sont introduites la diode électroluminescente et son électronique de commande, de la résine étant ensuite coulée dans le conteneur de façon à venir surmouler la plaquette de circuit imprimé sur laquelle sont montés les composants nécessaires au fonctionnement de la diode électroluminescente, cette dernière émergeant du conteneur.

Avantageusement, le tube métallique ou le conteneur dans lequel est introduit le module d'éclairage selon l'invention occupe un volume semblable à celui d'une ampoule à filament ordinaire, ce qui simplifie encore davantage le remplacement d'une telle ampoule à filament par la diode électroluminescente.

D'autres caractéristiques et avantages de la présente invention ressortiront plus clairement de la description détaillée qui suit d'un mode de réalisation du dispositif d'éclairage à diode électroluminescente selon l'invention, cet exemple étant donné à titre purement illustratif et non limitatif seulement en liaison avec le dessin annexé sur lequel :
- la figure 1 est une représentation schématique du module d'éclairage selon l'invention enfermé dans un volume clos ;
- la figure 2 est une vue analogue à celle de la figure 1 dans laquelle la source de courant est constituée par une source de tension extérieure au volume clos et qui alimente la diode électroluminescente via un convertisseur tension-courant ;
- la figure 3 est une vue analogue à celle de la figure 2 dans laquelle la source de tension extérieure est une source de tension variable, un dispositif de commande et de limitation du courant étant raccordé sur la source de courant afin de définir un rapport de proportionnalité entre la valeur de la tension appliquée et l'intensité du courant fourni par la source de courant qui détermine l'intensité de l'éclairage produit ;
- la figure 4 est une vue analogue à celle de la figure 3 dans laquelle le module d'éclairage comprend, entre la source de tension et la source de courant, un élément redresseur ;
- la figure 5 est une vue analogue à celle de la figure 4 dans laquelle un premier élément de protection contre les surtensions est intercalé entre l'élément redresseur et la source de courant, un second élément de protection en cas d'absence de charge étant disposé en aval de la source de courant ;
- la figure 6 est une vue analogue à celle de la figure 5 dans laquelle un premier filtre pour filtrer les interférences électromagnétiques générées par le fonctionnement de la pièce à main est intercalé entre l'élément redresseur et le premier élément de protection contre les surtensions, un second filtre ayant pour but de stabiliser et lisser le courant produit par la source de courant étant en aval de la source de courant, après l'élément de protection contre les absences de charge ;
- la figure 7 est une vue analogue à celle de la figure 1 dans laquelle la source de courant du module d'éclairage selon l'invention est alimentée depuis l'extérieur du volume clos par une source de courant externe et comprend des moyens pour faire varier l'intensité du courant afin d'obtenir le courant de fonctionnement de la diode électroluminescente ;
- les figures 8A à 8C sont des vues d'une première variante d'encapsulation du module d'éclairage selon l'invention dans laquelle le module d'éclairage est introduit dans un tube métallique, plastique ou céramique hermétiquement scellé;
- la figure 9 est une vue d'une seconde variante d'encapsulation du module d'éclairage selon l'invention dans laquelle le module d'éclairage est introduit dans un conteneur qui est rempli de résine, et
- la figure 10 est une représentation schématique d'une ampoule à filament du commerce qui permet d'apprécier que le module d'éclairage selon l'invention présente les mêmes formes et dimensions ainsi que le même agencement des connexions électriques qu'une telle ampoule.

La présente invention procède de l'idée générale inventive qui consiste à encapsuler dans un même volume clos au moins une diode électroluminescente et sa source de courant associée. On obtient ainsi un composant d'éclairage discret dont le fonctionnement est parfaitement indépendant des caractéristiques électriques de la pièce à main dans laquelle il est intégré. En outre, le composant discret peut remplacer pièce pour pièce une ampoule à filament du commerce sans qu'il soit nécessaire de modifier la pièce à main existante tant d'un point de vue mécanique que d'un point de vue électrique. Il est ainsi possible de porter les pièces à main existantes à un standard plus élevé tout en évitant à l'utilisateur le surcoût lié à l'achat d'une nouvelle pièce à main.

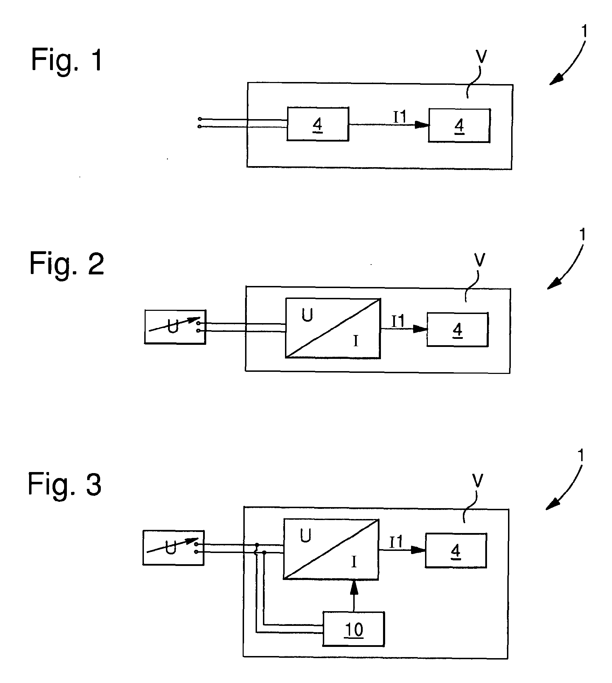

Désigné dans son ensemble par la référence numérique générale 1, le module d'éclairage selon la présente invention comprend (voir figure 1) une source de courant 2 délivrant un courant de fonctionnement l1 à une diode électroluminescente 4. Conformément à l'invention, la diode électroluminescente 4 et sa source de courant 2 associée sont intégrées dans un volume clos V comme il sera décrit plus en détail ultérieurement.

La source de courant 2 est alimentée depuis l'extérieur du volume clos V par une source de tension 6 et comprend des moyens 8 pour convertir la tension U fournie par la source de tension extérieure en courant de fonctionnement I1 de la diode électroluminescente (voir figure 2). Bien entendu, les moyens de conversion de tension 8 sont intégrés dans le même volume clos V que le module d'éclairage auquel ils appartiennent.

La source de tension 6 peut être une source de tension variable. Dans ce cas, on utilise une source de courant 2 dont le courant produit I1 varie en fonction de la tension d'entrée U. Ceci est nécessaire dans le cas où l'on souhaite que l'intensité de l'éclairage fourni par la diode 4 soit d'autant plus élevée que la tension appliquée U est grande. Dans le cas où la source de tension 6 utilisée est une source de tension variable, un dispositif 10 de commande et de limitation du courant est raccordé sur la source de courant 2 afin de définir un rapport de proportionnalité entre la valeur de la tension U appliquée et l'intensité du courant I1 fourni par la source de courant 2 qui détermine l'intensité de l'éclairage produit par la diode électroluminescente 4 (voir figure 3). Bien entendu, le dispositif 10 de commande et de limitation du courant est intégré dans le même volume clos V que la source de courant 2 et que la diode électroluminescente 4.

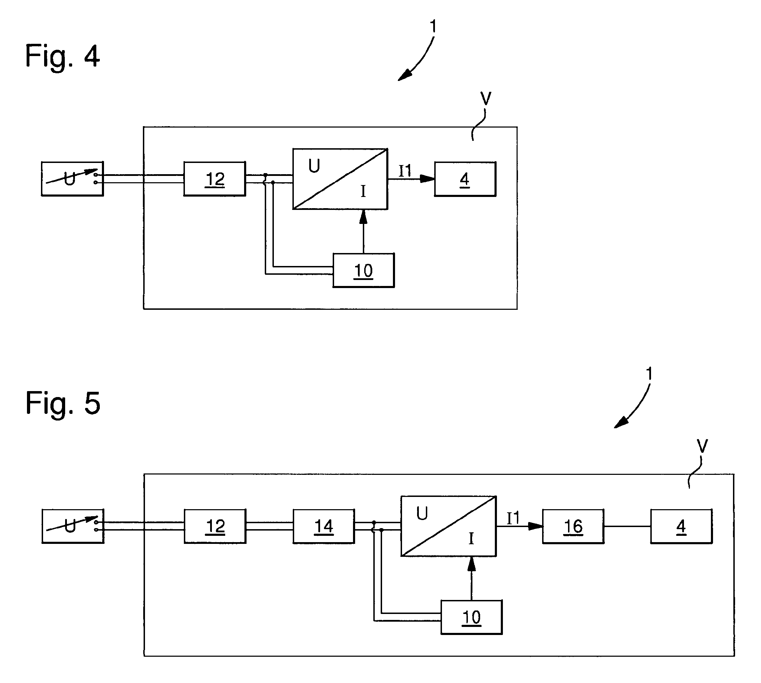

Selon une caractéristique complémentaire de l'invention, le module d'éclairage 1 comprend, entre la source de tension 6 et la source de courant 2, un élément redresseur 12 grâce auquel la diode électroluminescente 4 peut être montée sans considération de polarité (voir figure 4). L'élément redresseur 12 comprend un ensemble de quatre diodes montées en pont de Gretz qui agit de façon que, quelle que soit la polarité de la tension d'entrée U, la polarité de cette tension en sortie du pont de Gretz est toujours la même. Bien entendu, l'élément redresseur 12 est intégré dans le même volume clos V que le module d'éclairage 1 auquel il appartient.

Selon une autre caractéristique de l'invention, on intercale entre l'élément redresseur 12 et la source de courant 2 un premier élément de protection contre les surtensions 14 (voir figure 5). Ce premier élément de protection 14 a pour but de protéger le module d'éclairage 1 selon l'invention contre les surtensions qui peuvent survenir à l'entrée du module d'éclairage 1 en cas d'erreur d'utilisation de la part du praticien. En aval de la source de courant 2, on peut également prévoir un second élément de protection 16 en cas d'absence de charge. Ce second élément de protection 16 a pour but de protéger le module d'éclairage 1 en cas d'absence de la diode 4. En effet, si la diode 4 est manquante ou hors d'usage, la tension peut augmenter jusqu'à des valeurs correspondant à la destruction du module d'éclairage 1. Bien entendu, les premier et second modules de protection 14 et 16 sont intégrés dans le même volume clos V que le module d'éclairage 1 auquel ils appartiennent.

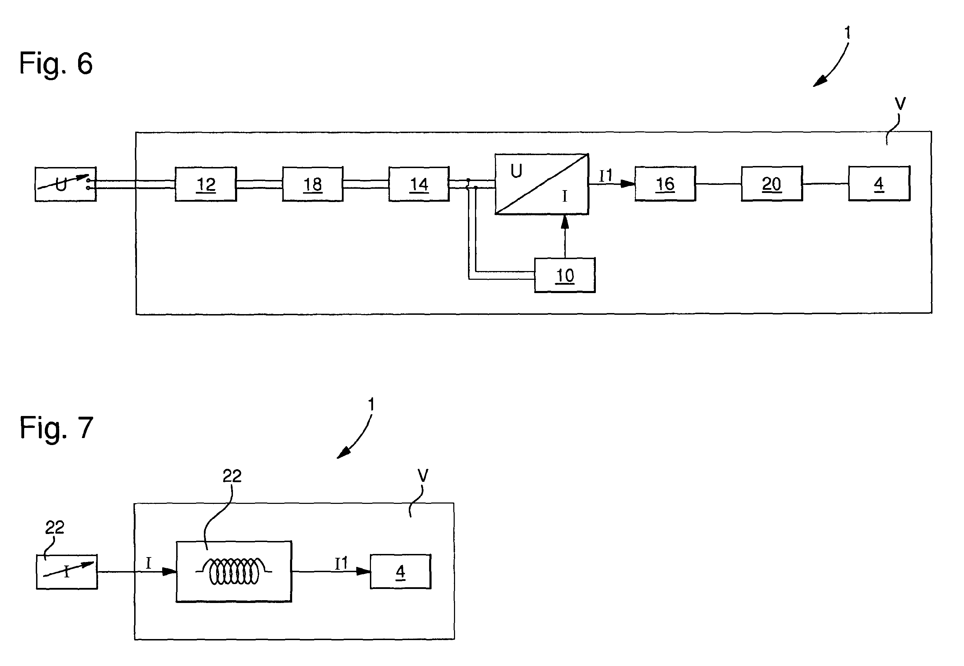

Selon encore une autre caractéristique de l'invention, on intercale entre l'élément redresseur 12 et le premier élément de protection 14 contre les surtensions un premier filtre 18 pour filtrer les interférences électromagnétiques générées par le fonctionnement de l'unité de commande et de fourniture de fluides à laquelle la pièce à main est raccordée et qui perturbent la source de courant 2 (voir figure 6). En aval de la source de courant 2, après l'élément de protection contre les absences de charge 16, on peut prévoir un second filtre 20 qui a pour but de stabiliser et lisser le courant produit par la source de courant 2 et de filtrer les interférences inhérentes au bruit provoqué par le fonctionnement de la source de courant 2. Effectivement, cette source de courant 2 est typiquement une source de courant à découpage fonctionnant à une fréquence de 1 Mhz. Bien entendu, les premier et second filtres 18 et 20 sont intégrés dans le même volume clos V que le module d'éclairage 1 auquel ils appartiennent.

Selon une seconde variante de réalisation de l'invention, la source de courant 2 du module d'éclairage 1 est alimentée depuis l'extérieur du volume clos V par une source de courant externe 22 délivrant un courant d'intensité I donnée et comprend des moyens 24 pour faire varier le courant d'intensité I donnée afin d'obtenir le courant de fonctionnement l1 de la diode électroluminescente 4 (voir figure 7). Bien entendu, les moyens de variation 24 de l'intensité du courant sont intégrés dans le même volume clos V que le module d'éclairage auquel ils appartiennent.

On s'intéresse maintenant à des techniques d'encapsulation du module d'éclairage 1 selon l'invention tel que décrit ci-dessus dans un volume clos V.

La diode électroluminescente 4 est un composant discret. Dans un mode de réalisation, elle comprend un élément actif 26 émetteur de lumière monté sur une première plaquette de circuit intégré 28 et relié aux pistes conductrices structurées sur cette plaquette par soudage par fil 30, technique mieux connue sous sa dénomination anglo-saxonne « wire bonding ». Bien entendu, cet exemple de réalisation d'une diode électroluminescente est donné à titre purement illustratif seulement et d'autres types de diodes électroluminescentes peuvent être envisagés par l'homme du métier sans sortir du cadre de l'invention tel que défini par les revendications annexées.

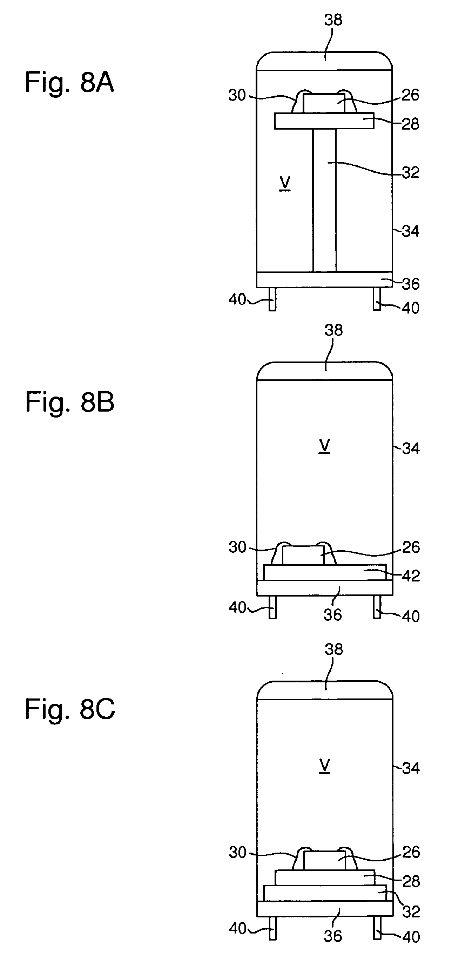

Les autres composants qui entrent dans la réalisation du module d'éclairage 1 selon l'invention sont montés sur une seconde plaquette de circuit imprimé 32 qui est par exemple fixée sous la première plaquette de circuit imprimé 28, perpendiculairement à cette dernière. L'ensemble ainsi formé peut être introduit dans un tube 34, par exemple métallique, plastique ou céramique, qui définit un volume hermétiquement clos V (voir figure 8A). Le tube 34 est fermé à sa base par une embase 36 et comprend à son extrémité supérieure, du côté de la diode électroluminescente 4, une lentille de collimation 38 collée ou sertie sur le tube 34. Un tel montage est stérilisable. Le montage est complété par deux pattes de connexion 40 qui font saillie du tube 34 à l'arrière de celui-ci et qui permettent le raccordement du module d'éclairage 1 à un réseau extérieur d'alimentation en électricité.

Selon une première variante de réalisation (figure 8B), la diode électroluminescente 4 et les composants électroniques nécessaires au fonctionnement de la diode 4 sont montés sur la même plaquette de circuit imprimé 42.

Selon une seconde variante de réalisation (figure 8C), la première plaquette de circuit imprimé 28 sur laquelle est fixée la diode électroluminescente 4 est assemblée en parallèle avec la seconde plaquette de circuit imprimé 32 sur laquelle sont montés les composants nécessaires au fonctionnement de la diode 4.

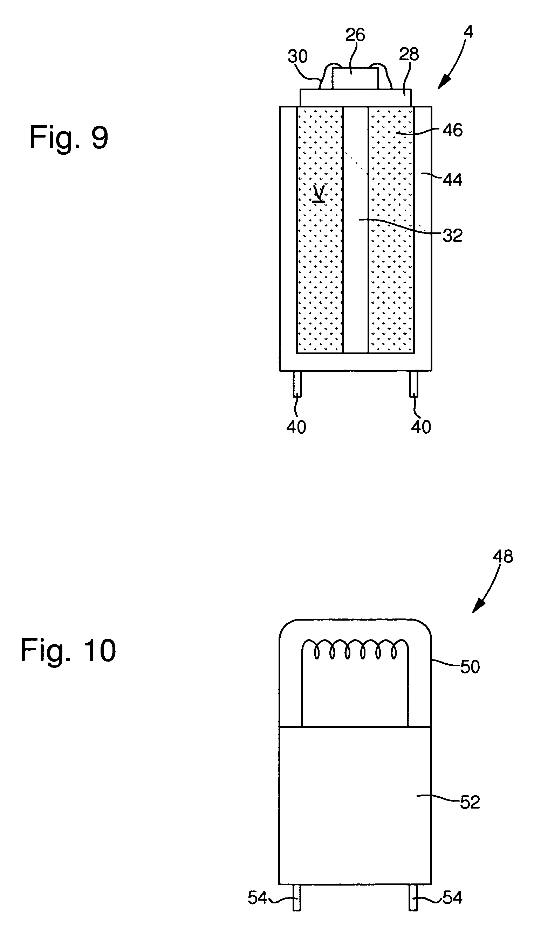

Une autre technique d'encapsulation du module d'éclairage 1 selon l'invention consiste (voir figure 9) à se munir d'un conteneur 44 dans lequel sont introduites la diode électroluminescente 4 et son électronique de commande. De la résine 46 est ensuite coulée dans le conteneur 44 de façon à venir surmouler la seconde plaquette de circuit imprimé 32 sur laquelle sont montés les composants nécessaires au fonctionnement de la diode électroluminescente 4, cette dernière émergeant du conteneur 44. Deux pattes de connexion 40 font saillie du conteneur 44 à l'arrière de celui-ci et permettent le branchement du module d'éclairage 1 à un réseau extérieur d'alimentation en électricité.

La figure 10 est une représentation schématique d'une ampoule à filament 48 du marché utilisée dans une pièce à main existante. Une telle ampoule comprend essentiellement un bulbe en verre 50 prolongé par un culot 52 et deux pattes de connexion 54 pour son branchement à un réseau extérieur d'alimentation en électricité. On appréciera l'identité de formes et de dimensions entre l'ampoule à filament 48 et le module d'éclairage 1 selon l'invention dont deux modes de réalisation sont illustrés à titre d'exemple aux figures 8 et 9. L'interchangeabilité entre l'ampoule à filament 48 et le module d'éclairage 1 est rendue totale par le fait que les pattes de connexion 40 du module d'éclairage 1 sont agencées de la même manière que les pattes de connexion 54 de l'ampoule 48.

La présente invention procure ainsi un module d'éclairage à diode électroluminescente constituant un élément consommable amovible dans le volume intérieur duquel sont logés de manière indissociable tous les composants électroniques nécessaires au fonctionnement de la diode. Il est ainsi possible de porter une pièce à main existante à un standard de fonctionnement plus élevé en remplaçant une ampoule à filament du marché par un composant à diode électroluminescente. L'utilisateur dispose ainsi d'une pièce à main équipée d'un dispositif d'éclairage dont la durée de vie est plus longue et qui produit une lumière très proche de celle de la lumière naturelle, sans pour autant être dans l'obligation de faire l'acquisition d'une pièce à main neuve, ni même de devoir adapter d'un point de vue mécanique ou électrique la pièce à main qui est en sa possession. Ce résultat remarquable est atteint grâce au fait que le module d'éclairage selon l'invention renferme dans son volume intérieur tous les composants électroniques nécessaires au bon fonctionnement de la diode, ce qui permet d'éviter de devoir modifier l'agencement électrique de la pièce à main et/ou de l'unité de commande et de fourniture de fluides à laquelle la pièce à main est raccordée, et grâce au fait que le module d'éclairage selon l'invention présente des caractéristiques géométriques et de connexion électrique identiques à celles de l'ampoule à filament qu'il remplace, ce qui permet d'éviter de devoir modifier l'agencement mécanique de la pièce à main.

## Revendications

1. Module d'éclairage à diode électroluminescente pour pièce à main à usage dentaire ou chirurgical, ce module d'éclairage (1) comprenant une source de courant (2) délivrant un courant de fonctionnement (11) à une diode électroluminescente (4), **caractérisé en ce que** la source de courant (2) et la diode électroluminescente (4) sont encapsulées dans un même volume clos (V), le module d'éclairage (1) comprenant des moyens de connexion électriques pour son raccordement à un réseau d'alimentation en électricité.

2. Module d'éclairage selon la revendication 1, **caractérisé en ce que** le module d'éclairage (1) constitue une unité interchangeable.

3. Module d'éclairage selon l'une quelconque des revendications 1 ou 2, **caractérisé en ce que** la source de courant (2) est alimentée depuis l'extérieur du volume clos (V) par une source de tension (6) et comprend des moyens (8) pour convertir la tension (U) fournie par la source de tension extérieure (6) en courant de fonctionnement (11) de la diode électroluminescente (4).

4. Module d'éclairage selon la revendication 3, **caractérisé en ce que** la source de tension (6) est une source de tension variable et **en ce que** la source de courant (2) produit un courant qui varie en fonction de la tension fournie par la source de tension variable (6).

5. Module d'éclairage selon la revendication 4, **caractérisé en ce qu'**un dispositif (10) de commande et de limitation du courant est raccordé sur la source de courant (2) afin de définir un rapport de proportionnalité entre la valeur de la tension (U) fournie par la source de tension variable (6) et l'intensité du courant (I1) fourni par la source de courant (2) qui conditionne l'intensité de l'éclairage produit par la diode électroluminescente (4).

6. Module d'éclairage selon l'une quelconque des revendications 1 à 5, **caractérisé en ce que** le module d'éclairage (1) comprend, en amont de la source de courant (2), un élément redresseur (12) grâce auquel la diode électroluminescente (4) peut être montée sans considération de polarité.

7. Module d'éclairage selon la revendication 6, caractérisé en ce le module d'éclairage (1) comprend un premier élément de protection contre les surtensions (14) intercalé entre l'élément redresseur (12) et la source de courant (2), ce premier élément de protection (14) protégeant le module d'éclairage (1) contre les surtensions qui surviennent à l'entrée du module d'éclairage (1).

8. Module d'éclairage selon la revendication 7, **caractérisé en ce que** le module d'éclairage (1) comprend, en aval de la source de courant (2), un second élément de protection (16) contre les surtensions qui proviennent en cas d'absence de la diode électroluminescente (4).

9. Module d'éclairage selon la revendication 8, **caractérisé en ce que** le module d'éclairage (1) comprend, intercalé entre l'élément redresseur (12) et le premier élément de protection contre les surtensions (14) un premier filtre (18) pour filtrer les interférences électromagnétiques qui perturbent la source de courant (2).

10. Module d'éclairage selon la revendication 9, **caractérisé en ce que** le module d'éclairage (1) comprend, après le second élément de protection (16) contre les surtensions, un second filtre (20) qui filtre les interférences inhérentes au bruit provoqué par le fonctionnement de la source de courant (2).

11. Module d'éclairage selon l'une quelconque des revendications 6 à10, **caractérisé en ce que** la source de courant (2) est alimentée depuis l'extérieur du volume clos (V) par une source de courant externe (22) délivrant un courant d'intensité (I) donnée et comprend des moyens pour faire varier le courant d'intensité (I) donnée afin d'obtenir le courant de fonctionnement (I1) de la diode électroluminescente (4).

12. Module d'éclairage selon l'une quelconque des revendications 1 à 11, **caractérisé en ce que** le module d'éclairage (1) et la diode électroluminescente (4) sont introduits dans un tube métallique, plastique ou céramique (34) qui définit un volume (V) hermétiquement clos.

13. Module d'éclairage selon la revendication 12, **caractérisé en ce que** le tube (34) est fermé à sa base par une embase (36) et comprend à son extrémité supérieure, du côté de la diode électroluminescente (4), une lentille de collimation (38) collée ou sertie sur le tube (34).

14. Module d'éclairage selon l'une quelconque des revendications 1 à 11, **caractérisé en ce que** le module d'éclairage (1) et la diode électroluminescente (4) sont introduits dans un conteneur (44) dans lequel est coulée une résine (46) de façon à venir surmouler le module d'éclairage (1), la diode électroluminescente (4) émergeant du conteneur (44).

15. Module d'éclairage selon l'une quelconque des revendications 12 à 14, **caractérisé en ce que** le module d'éclairage (1) est formé par une plaquette de circuit imprimé (32) sur laquelle sont montés les composants nécessaires au fonctionnement de la diode électroluminescente (4).

## Patentansprüche

1. Beleuchtungsmodul mit lichtemittierender Diode für ein dentales oder chirurgisch verwendbares Handstück, wobei das Beleuchtungsmodul (1) eine Stromquelle (2) aufweist, die einen Betriebsstrom (I1) an eine lichtemittierende Diode (4) liefert, **dadurch gekennzeichnet, dass** die Stromquelle (2) und die lichtemittierende Diode (4) in einem gemeinsamen geschlossenen Volumen (V) eingekapselt sind und wobei das Beleuchtungsmodul (1) elektrische Verbindungsmittel für seine Verbindung an ein elektrisches Versorgungsnetz aufweist.

2. Beleuchtungsmodul nach Anspruch 1, **dadurch gekennzeichnet, dass** das Beleuchtungsmodul (1) eine austauschbare Einheit bildet.

3. Beleuchtungsmodul nach einem der Ansprüche 1 oder 2, **dadurch gekennzeichnet, dass** die Stromquelle (2) von außerhalb des geschlossenen Volumens (V) von einer Spannungsquelle (6) versorgt wird und Mittel (8) zum Konvertieren der Spannung (U), welche von der externen Spannungsquelle (6) geliefert wird, in einen Betriebsstrom (I1) der lichtemittierenden Diode (4) aufweist.

4. Beleuchtungsmodul nach Anspruch 3, **dadurch gekennzeichnet, dass** die Spannungsquelle (6) eine variable Spannungsquelle ist und dass die Stromquelle (2) einen Strom produziert, welcher als Funktion der von der variablen Spannungsquelle (6) gelieferten Spannung variiert.

5. Beleuchtungsmodul nach Anspruch 4, **dadurch gekennzeichnet, dass** eine Vorrichtung (10) zur Steuerung und zur Begrenzung des Stroms mit der Stromquelle (2) verbunden ist, um ein proportionales Verhältnis zwischen dem Spannungswert (U), welcher von der variablen Spannungsquelle (6) geliefert wird und der Stromstärke (I1), welche von der Stromquelle (2) geliefert wird, zu bilden, dass die Intensität der von der lichtemittierenden Diode (4) erzeugten Beleuchtung bestimmt.

6. Beleuchtungsmodul nach einem der Ansprüche 1 bis 5, **dadurch gekennzeichnet, dass** das Beleuchtungsmodul (1) stromaufwärts der Stromquelle (2) ein Gleichrichterelement (12) aufweist, mittels welchem die lichtemittierende Diode (4) ohne Rücksicht auf ihre Polarität montiert werden kann.

7. Beleuchtungsmodul nach Anspruch 6, **dadurch gekennzeichnet, dass** das Beleuchtungsmodul (1) ein erstes Schutzelement gegen Überspannungen (14) aufweist, welches zwischen dem Gleichrichterelement (12) und der Stromquelle (2) angeordnet ist, wobei das erste Schutzelement (14) das Beleuchtungsmodul (1) gegen Überspannungen schützt, die am Eingang des Beleuchtungsmoduls (1) auftreten.

8. Beleuchtungsmodul nach Anspruch 7, **dadurch gekennzeichnet, dass** das Beleuchtungsmodul (1) stromabwärts der Stromquelle (2) ein zweites Schutzelement (16) gegen Überspannungen aufweist, die im Falle des Fehlens der lichtemittierenden Diode (4) auftreten.

9. Beleuchtungsmodul nach Anspruch 8, **dadurch gekennzeichnet, dass** das Beleuchtungsmodul (1) einen ersten Filter (18) zwischen dem Gleichrichterelement (12) und dem ersten Schutzelement gegen Überspannungen (14) aufweist, um elektromagnetische Interferenzen zu filtern, die die Stromquelle (2) stören.

10. Beleuchtungsmodul nach Anspruch 9, **dadurch gekennzeichnet, dass** das Beleuchtungsmodul (1) nach dem zweiten Schutzelement (16) gegen Überspannungen einen zweiten Filter (20) aufweist, welcher inhärente Interferenzen des Rauschens filtert, die durch den Betrieb der Stromquelle (2) hervorgerufen sind.

11. Beleuchtungsmodul nach einem der Ansprüche 6 bis 10, **dadurch gekennzeichnet, dass** die Stromquelle (2) von außerhalb des geschlossenen Volumens (V) von einer externen Stromquelle (22) versorgt ist, die einen Strom vorgegebener Stärke (I) liefert und die Mittel zum Variieren des Stroms vorgegebene Stärke (I) aufweist, um den Betriebsstrom (I1) für die lichtemittierende Diode (4) zu erhalten.

12. Beleuchtungsmodul nach einem der Ansprüche 1 bis 11, **dadurch gekennzeichnet, dass** das Beleuchtungsmodul (1) und die lichtemittierende Diode (4) in einem metallischen, Kunststoff- oder keramischen Rohr (34) eingeführt sind, welches ein hermetisch geschlossenes Volumen (V) definiert.

13. Beleuchtungsmodul nach Anspruch 12, **dadurch gekennzeichnet, dass** das Rohr (34) an seiner Basis durch einen Boden (36) verschlossen ist und an seinem oberen Ende, auf Seiten der lichtemittierenden Diode (4) eine Kollimationslinse (38) aufweist, die auf das Rohr (34) geklebt oder gequetscht ist.

14. Beleuchtungsmodul nach einem der Ansprüche 1 bis 11, **dadurch gekennzeichnet, dass** das Beleuchtungsmodul (1) und die lichtemittierende Diode (4) in einen Behälter (44) eingeführt sind, in welchem ein Harz (46) derart eingefüllt ist, um das Beleuchtungsmodul (1) zu umspritzen, wobei die lichtemittierende Diode (4) aus dem Behälter (44) herausragt.

15. Beleuchtungsmodul nach einem der Ansprüche 12 bis 14, **dadurch gekennzeichnet, dass** das Beleuchtungsmodul (1) von einer gedruckten Leiterplatine (32) gebildet ist, auf welcher die zum Betrieb der lichtemittierenden Diode (4) notwendigen Bauteile montiert sind.

## Claims

1. Lighting module with an LED for a handpiece for dental or surgical use, said lighting module (1) including a current source (2) delivering an operating current (I1) to an LED (4), **characterized in that** the current source (2) and the LED (4) are encapsulated in the same enclosed volume (V), the lighting module (1) including electrical connection means for the connection thereof to an electrical power grid.

2. Lighting module according to claim 1, **characterized in that** the lighting module (1) forms a unit that can be interchanged.

3. Lighting module according to any of claims 1 or 2, **characterized in that** the current source (2) is powered from outside the enclosed volume (V) by a voltage source (6) and includes means (8) for converting the voltage (U) supplied by the external voltage source (6) into operating current (I1) for the LED (4).

4. Lighting module according to claim 3, **characterized in that** the voltage source (6) is a variable voltage source and **in that** the current source (2) produces a current that varies according to the voltage supplied by the variable voltage source (6).

5. Lighting module according to claim 5, **characterized in that** a current control and limiting device (10) is connected across the current source (2) so as to define a ratio of proportionality between the value of the voltage (U) supplied by the variable voltage source (6) and the intensity of the current (I1) supplied by the current source (2), which determines the lighting intensity produced by the LED (4).

6. Lighting module according to any of claims 1 to 5, **characterized in that**, upstream of the current source (2), the lighting module (1) includes a rectifier element (12) allowing the LED (4) to be arranged regardless of polarity.

7. Lighting module according to claim 6, **characterized in that** the lighting module (1) includes a first overvoltage protection element (14) inserted between the rectifier element (12) and the current source (2), said first protection element (14) protecting the lighting module (1) against any overvoltage that may occur at the input of the lighting module (1).

8. Lighting module according to claim 7, **characterized in that**, downstream of the current source (2), the lighting module (1) includes a second protection element (16) against any overvoltage that occurs in the absence of the LED (4).

9. Lighting module according to claim 8, **characterized in that**, inserted between the rectifier element (12) and the first overvoltage protection element (14), the lighting module (1) includes a first filter (18) for filtering any electromagnetic interference that disturbs the current source (2).

10. Lighting module according to claim 9, **characterized in that**, after the second overvoltage protection element (16), the lighting module (1) includes a second filter (20), which filters the interference inherent to the noise caused by the operation of the current source (2).

11. Lighting module according to any of claims 6 to 10, **characterized in that** the current source (2) is powered from outside the enclosed volume (V) by an external current source (22) delivering a current of given intensity (I) and includes means for varying the current of given intensity (I) so as to obtain the operating current (I1) for the LED (4).

12. Lighting device according to any of claims 1 to 11, **characterized in that** the lighting module (1) and the LED (4) are inserted into a metal, plastic or ceramic tube (34) which defines a hermetically sealed volume (V).

13. Lighting module according to claim 12, **characterized in that** the tube (34) is closed at the base thereof by an end piece (36) and includes at the top end thereof, on the side of the LED (4), a collimation lens (38) which is bonded or crimped onto the tube (34).

14. Lighting module according to any of claims 1 to 11, **characterized in that** the lighting module (1) and the LED (4) are placed in a container (44) into which a resin (46) is poured so as to encapsulate the lighting module (1), with the LED (4) emerging from the container (44).

15. Lighting module according to any of claims 12 to 14, **characterized in that** the lighting module (1) is formed by a printed circuit board (32) on which the components necessary for the operation of the LED (4) are mounted.
